# EUROPÄISCHE PATENTSCHRIFT

(11) **EP 2 813 252 B1**
(45) Veröffentlichungstag und Bekanntmachung des Hinweises auf die Patenterteilung: **09.08.2017**
(21) Anmeldenummer: 14172305.6
(22) Anmeldetag: 13.06.2014
(51) Int. Cl.: A61M 1/16, A61M 1/36, B01D 61/24, B01D 61/30, B01D 63/02, B01D 63/08

(54) **Filtervorrichtung für Blut und dergleichen Körperflüssigkeiten**
Filter device for blood and similar body fluids
Dispositif de filtre pour le sang et liquides corporels

(30) Priorität: 14.06.2013 DE 102013106247
(43) Veröffentlichungstag der Anmeldung: 17.12.2014
(73) Patentinhaber: B. Braun Avitum AG, 34212 Melsungen (DE)
(72) Erfinder: Dr. Martins, Konrad, 01277 Dresden (DE)
(74) Vertreter: Winter, Brandl, Fürniss, Hübner, Röss, Kaiser, Polte - Partnerschaft mbB

(56) Entgegenhaltungen:
- EP-A2- 1 110 563
- EP-A2- 1 913 964
- DE-A1- 2 716 178
- DE-A1- 2 749 516
- US-A- 3 880 759
- US-A- 4 108 764

## Beschreibung

Die Erfindung betrifft eine Filtervorrichtung zur selektiven Entfernung von Substanzen aus Blut oder anderen Körperflüssigkeiten, insbesondere eine Dialysefiltervorrichtung, sowie Verfahren zum Befüllen der Filtervorrichtung mit einer wässrigen Lösung.

Filtervorrichtungen zur selektiven Entfernung von Substanzen aus Blut oder anderen Körperflüssigkeiten können zum Beispiel Dialysefiltervorrichtungen, auch Dialysatoren genannt, sein. Sie weisen im Allgemeinen zwei durch eine semipermeable Membran getrennte Kompartimente auf, beispielsweise ein Blutkompartiment (blutführendes Kompartiment) und ein Dialysatkompartiment (dialysatführendes Kompartiment). Im Einsatz bei einem Dialyseverfahren strömt Blut bzw. die zu reinigende (filtrierende) Körperflüssigkeit durch das Blutkompartiment und - in der Regel im Gegenstrom dazu - die Dialysierflüssigkeit (Dialysat) durch das Dialysatkompartiment. Über die semipermeable Membran erfolgt ein Stofftransport bestimmter Stoffe zwischen dem Blutkompartiment und dem Dialysatkompartiment durch Diffusion oder Konvektion.

Die am häufigsten eingesetzte Bauform eines Dialysators ist ein Kapillardialysator, der in einem Gehäuse eine große Anzahl an Hohlfasern aufweist. In den Hohlfasern wird das Blut bzw. die zu reinigende Körperflüssigkeit geführt, während der Außen/Umgebungsraum der Fasern von der Dialysierflüssigkeit durchströmt wird, d.h. die Hohlfasern außenseitig von der Dialysierflüssigkeit umströmt werden. Hierbei dienen die Wände der Hohlfasern als semipermeable Membran. Daneben gibt es auch Plattendialysatoren mit schichtförmigen Membranen.

Ein weiteres Beispiel einer Filtervorrichtung zur selektiven Entfernung von Substanzen aus Blut oder anderen Körperflüssigkeiten ist eine Adsorberkartusche, wie sie zum Beispiel bei der Hämoperfusion verwendet werden kann. Hierbei wird die zu reinigende Körperflüssigkeit durch ein in der Adsorberkartusche enthaltenes Adsorbens gepumpt, das bestimmte zu entfernende Substanzen adsorbieren kann und somit aus dem Blut oder der zu reinigenden Körperflüssigkeit entfernen kann.

Allen diesen Filtervorrichtungen ist gemein, dass die semipermeable Membran bzw. das Adsorbens eine große spezifische Oberfläche aufweist, um einen effektiven Stofftransport und somit eine effektive Reinigung bzw. Filtration zu gewährleisten.

Die Hämokompatibilität und die Leistungsfähigkeit derartiger Filtervorrichtungen hängen unter anderem wesentlich davon ab, in welchem Ausmaß Luft aus der Filtervorrichtung vor deren Einsatz bei der gewünschten medizinischen Behandlung entfernt wurde. Dies hängt zum einen damit zusammen, dass Luft in dem Blutkompartiment das Gerinnungsrisiko durch Blut-Luft-Kontakt erhöhen kann und sogar bei der Rückführung des gereinigten Blutes in den Körper die Gefahr von Luftinfusionen, die zu Luftembolien führen können, mit sich bringen kann. Zum anderen tragen die Bereiche der Membranoberflächen bzw. Adsorberoberflächen, die auf Grund von Luftansammlungen nicht mit Flüssigkeit in Kontakt stehen, nicht zum Stofftransport bei, wodurch die Leistungsfähigkeit der Filtervorrichtung verringert wird.

Zur Entlüftung wird herkömmlicherweise versucht, die Filtervorrichtung von unten nach oben mit einer wässrigen Lösung zu befüllen, so dass die Luft nach Möglichkeit am oberen Ende der Filtervorrichtung entweichen kann, und anschließend verbleibende Luftansammlungen durch Drehen und Klopfen zu entfernen. Als Beispiel hierfür wird auf die DE 10 2008 045 422 A1 verwiesen, wobei die dort beschriebene Filtervorrichtung zusätzlich Mittel zum Bestimmen des Füllungszustandes vorsieht.

Diese in der Regel manuelle Vorgehensweise ist allerdings zeitraubend für den Benutzer. Ein automatisiertes Verfahren zum Spülen bzw. Befüllen einer Blutbehandlungsvorrichtung ist in der DE 10 2011 102 492 A1 beschrieben, wobei verschiedene Flussraten und Drücke zur Entlüftung angelegt werden.

Allerdings wäre es wünschenswert, die Befüllung von Filtervorrichtungen für die Benutzer, in der Regel das Krankenhauspersonal, zu erleichtern. Daneben stellt insbesondere die hohe spezifische Membran- bzw. Adsorberoberfläche der Filtervorrichtungen, an die sich bevorzugt kleine und kleinste Luftblasen anlagern können, ein Problem bei der möglichst vollständigen Entfernung von Luft dar, das durch Drehen und Klopfen alleine meist nicht zufriedenstellend gelöst werden kann.

In der EP 1 913 964 A2 wird eine Hohlfaserfiltervorrichtung zur Reinigung von Blut offenbart, deren Hohlfasermembrane aus Polysulfon-Harz und Polyvinylpyrrolidon bestehen und mit einem Radikalenfängermaterial beschichtet sind. Das Polyvinylpyrrolidon ist aufgrund seiner Hydrophilie vorgesehen, um die Kompatibilität der Membrane mit Blut zu verbessern.

In der EP 1 110 563 A2 wird eine Dialysevorrichtung beschrieben, welche eine semipermeable Membran mit einem hydrophoben Polymer und einem hydrophilen Polymer aufweist.

Gattungsgemäße Filtervorrichtungen sind des Weiteren in der US 3,880,759 A und der US 4,108,764 A beschrieben.

Die DE 27 16 178 A1 beschreibt ein Verfahren zur Herstellung einer gasimpermeablen Packung, die einen Nierendialysator und eine kontrollierte Atmosphäre enthält. Bei diesem Verfahren wird ein sterilisierter Dialysator in einer offen gasimpermeablen Umhüllung vorgesehen, der Dialysator und die Umhüllung wird evakuiert, eine kontrollierte Atmosphäre wird in die Umhüllung und den Dialysator geleitet und die Umhüllung wird unter Bildung einer gasimpermeablen Packung abgedichtet.

Die DE 27 49 516 A1 betrifft ein Verfahren zur Wärmesterilisierung von künstlichen Nieren, die einen Aufnahmebehälter mit darin untergebrachten, durchlässigkeitsselektiven Hohlfäden aufweist. Bei diesem Verfahren ist während der Wärmesterilisierung ein Druckausgleich vorgesehen, um Beschädigungen aufgrund thermischer Expansion zu vermeiden.

Aufgabe der vorliegenden Erfindung war es daher, eine Filtervorrichtung zur selektiven Entfernung von Substanzen aus Blut oder anderen Körperflüssigkeiten, insbesondere eine Dialysefiltervorrichtung, bereitzustellen, deren Entlüftung in einfacher und zuverlässiger Art und Weise durch den Benutzer möglich ist und eine möglichst vollständige Befüllung mit einer wässrigen Lösung ermöglicht.

Diese Aufgabe wird gelöst durch eine Filtervorrichtung zur selektiven Entfernung von Substanzen aus Blut oder anderen Körperflüssigkeiten, wobei die Filtervorrichtung mit einem Gas oder Gasgemisch gefüllt ist, das eine mindestens zweifach höhere Löslichkeit in Wasser als Luft aufweist.

Es wurde überraschenderweise gefunden, dass durch Austausch der Luft in der Filtervorrichtung durch ein Gas oder Gasgemisch, das eine mindestens zweifach höhere Löslichkeit in Wasser als Luft aufweist, beim Befüllen der Filtervorrichtung mit einer wässrigen Lösung verbleibende Gas- oder Gasgemischansammlungen sich verstärkt in der wässrigen Flüssigkeit lösen und sich vollständig entfernen lassen. Hierdurch kann eine vollständig entlüftete bzw. vollständig befüllte Filtervorrichtung auf einfache Weise erhalten werden, so dass die eingangs beschriebenen Probleme, die Luftrückstände bei der Behandlung bereiten können, vermieden werden können. Eine Befüllung der Filtervorrichtung ist durch den Benutzer durch einfaches Spülen der mit dem Gas oder Gasgemisch gefüllten Filtervorrichtung mit einer wässrigen Lösung möglich, ohne dass es ein manuelles Eingreifen, wie ein Klopfen an oder ein Drehen der Filtervorrichtung, bedarf.

Dementsprechend betrifft die vorliegende Erfindung weiterhin ein Verfahren zum Befüllen einer Filtervorrichtung für Blut oder andere Körperflüssigkeiten mit einer wässrigen Lösung, bei dem eine erfindungsgemäße Filtervorrichtung mit der wässrigen Lösung befüllt wird. Die Erfindung ist definiert durch die Merkmale der unabhängigen Ansprüche 1 und 8.

Jegliche Offenbarung im Zusammenhang mit der erfindungsgemäßen Filtervorrichtung gilt auch für das erfindungsgemäße Verfahren zum Befüllen der Filtervorrichtung, ebenso wie jegliche Offenbarung im Zusammenhang mit dem erfindungsgemäßen Verfahren zum Befüllen der Filtervorrichtung auch für die erfindungsgemäße Filtervorrichtung gilt.

Die Filtervorrichtung zur selektiven Entfernung von Substanzen aus Blut oder anderen Körperflüssigkeiten kann insbesondere eine Dialysefiltervorrichtung bzw. ein Dialysator sein. Die Filtervorrichtung weist insbesondere ein Gehäuse auf, das mindestens eine semipermeable Membran enthält und zwei durch die Membran getrennte Kompartimente, beispielsweise ein Blutkompartiment (blutführendes Kompartiment) und ein Dialysatkompartiment (dialysatführendes Kompartiment), sowie die entsprechenden Anschlüsse für den Zufluss und Abfluss des Blutes bzw. der zu reinigenden Körperflüssigkeit sowie des Dialysats. Insbesondere kann es sich bei der Filtervorrichtung um ein Hohlfaser-Membranmodul oder einen Hohlfaserdialysator, wie er beispielweise in der EP 1 714 692 A1 beschrieben ist. Es kann sich bei der Filtervorrichtung auch um eine Adsorberkartusche handeln, wie sie beispielweise bei der Hämoperfusion verwendet werden kann, oder um eine sonstige Filtervorrichtung handeln, die zur selektiven Entfernung von Substanzen aus Blut oder Blutbestandteilen oder sonstigen Körperflüssigkeiten geeignet ist.

Das Gas oder Gasgemisch, mit der die Filtervorrichtung gefüllt ist, weist eine mindestens zweifach höhere Löslichkeit in Wasser als Luft auf. Bei der Löslichkeit handelt es sich insbesondere um die Löslichkeit bei 25 °C und 1013 mbar. Die Löslichkeit von Gasen in Wasser kann auch mittels Henry-Konstanten gekennzeichnet werden. Beispielsweise beträgt die dimensionslose Henry-Konstante k_{H,cc}, die sich aus dem Quotienten der Konzentration des Gases in Wasser (in mol/L) durch die Konzentration des Gases in der Gasphase (in mol/L) bestimmt, für N₂, O₂ bzw. Ar, mit ca. 78,08 Vol.-%, 20,94 Vol.-% bzw. 0,93 Vol.-% die Hauptbestandteile der Luft, 1,492 x 10⁻², 3,180 x 10⁻² bzw. 3,425 x 10⁻² bei 25 °C in Wasser.

Das Gas oder Gasgemisch kann insbesondere eine dreifach höhere Löslichkeit in Wasser als Luft aufweisen, vorzugsweise eine vierfach höhere Löslichkeit in Wasser als Luft, stärker bevorzugt eine fünffach höhere Löslichkeit in Wasser als Luft, stärker bevorzugt eine zehnfach höhere Löslichkeit in Wasser als Luft, stärker bevorzugt eine fünfzehnfach höhere Löslichkeit in Wasser als Luft und insbesondere eine zwanzigfach höhere Löslichkeit in Wasser als Luft.

Hierbei kann es sich bei dem Gas insbesondere um CO₂ handeln bzw. das Gasgemisch kann CO₂ umfassen, insbesondere eine im Vergleich zu Luft erhöhte Konzentration an CO₂ aufweisen. Üblicherweise beträgt die Konzentration an CO₂ in der Luft ca. 0,04 Vol.-%. Die dimensionslose Henry-Konstante k_{H,cc} beträgt für CO₂ 0,832 bei 25 °C in Wasser. Diese hohe Löslichkeit von CO₂ in Wasser hängt insbesondere mit dessen Fähigkeit zur Bildung von Kohlensäure H₂CO₃ in Wasser und dessen unmittelbarer Dissoziation unter Bildung von Hydrogencarbonat oder Carbonat. Dementsprechend kann das Gas oder Gasgemisch auch andere, zur Dissoziation in Wasser fähige Gase aufweisen.

Das Gas oder Gasgemisch enthält vorzugsweise mindestens 50 Vol.-% CO₂, stärker bevorzugt mindestens 60 Vol.-% CO₂, stärker bevorzugt mindestens 70 Vol.-% CO₂, stärker bevorzugt mindestens 75 Vol.-% CO₂, stärker bevorzugt mindestens 80 Vol.-% CO₂, stärker bevorzugt mindestens 85 Vol.-% CO₂ stärker bevorzugt mindestens 90 Vol.-% CO₂, stärker bevorzugt mindestens 95 Vol.-% CO₂, stärker bevorzugt mindestens 98 Vol.-% CO₂, insbesondere besteht das Gas oder Gasgemisch aus CO₂ und ggf. unvermeidbaren Verunreinigungen.

Der mit dem Gas oder Gasgemisch gefüllte Innenraum der Filtervorrichtung ist an der Innenseite des Gehäuses, vorzugsweise im Bereich des reinigungsflüssigkeitsseitigen Eingangs und/oder Ausgangs der Filtervorrichtung , zusätzlich mit einem hydrophilen Material zumindest teilweise versehen, um die Reaktionsfläche zur Lösung des Gases zu vergrößern. In anderen Worten ausgedrückt ist der mit dem Gas oder Gasgemisch gefüllte Innenraum der Filtervorrichtung an der Innenseite des Gehäuses der Filtervorrichtung an der Grenzfläche zur Gas- oder Gasgemischatmosphäre zumindest teilweise mit einem wasserbenetzbaren (hydrophilen) Material versehen. Hierdurch kann die Ausspülbarkeit des Gases oder Gasgemisches mit einer wässrigen Lösung weiter verbessert werden.

In einer Weiterbildung weist die Filtervorrichtung gasdichte Verschlüsse auf oder sie ist gasdicht verpackt, vorzugsweise beides. Ebenso ist es vorteilhaft, wenn die Filtervorrichtung steril vorliegt. Hierbei kann das Gas oder Gasgemisch vor der Sterilisation in die Filtervorrichtung eingebracht werden, die Filtervorrichtung durch gasdichte Verschlüsse verschlossen und/oder in eine gasdichte Verpackung gegeben werden und anschließend sterilisiert werden, so dass das Gas oder Gasgemisch bis unmittelbar vor der Anwendung in der Filtervorrichtung gehalten werden kann und dem Benutzer, in der Regel dem Krankenhauspersonal, ein einfaches Befüllen bzw. Entlüften der Filtervorrichtung ermöglicht wird.

Es ist vorteilhaft, wenn ferner die semipermeable Membran zumindest teilweise mit einem wasserbenetzbaren (hydrophilen) Material versehen ist, das vorzugsweise fest, insbesondere kovalent, beispielsweise durch Aufpfropfen an die Membran oder die innere Oberfläche des Gehäuses, an die entsprechenden Teilen der Filtervorrichtung gebunden ist.

Insbesondere kann der mit dem Gas oder Gasgemisch gefüllte Innenraum der Filtervorrichtung an der Grenzfläche zur Gas- oder Gasgemischatmosphäre zu mindestens 5 Flächen-%, insbesondere mindestens 10 Flächen-%, insbesondere mindestens 20 Flächen-%, insbesondere mindestens 30 Flächen-%, und zu maximal 80 Flächen-%, insbesondere maximal 70 Flächen-%, insbesondere maximal 60 Flächen%, insbesondere maximal 50 Flächen-% mit einem wasserbenetzbaren Material versehen sein.

Bei dem wasserbenetzbaren Material kann es sich insbesondere um ein hydrophiles Material bzw. um ein Hydrophilisierungsmittel handeln. Beispiele hierfür sind Polyvinylpyrrolidon (PVP), Poly(meth)acrylate und Polyalkylenoxide, insbesondere Polyethylenglykol (PEG) und Polypropylenglykol, oder Kombinationen hiervon.

Die vorliegende Erfindung betrifft ferner ein Verfahren zum Befüllen einer Filtervorrichtung für Blut oder andere Körperflüssigkeiten mit einer wässrigen Lösung, bei dem eine erfindungsgemäße Filtervorrichtung mit der wässrigen Lösung befüllt wird.

Insbesondere kann während des Befüllens der Filtervorrichtung mit dem Gas oder Gasgemisch und/oder während des Befüllens der Filtervorrichtung mit der wässrigen Lösung ein Überdruck von mindestens 0,2 bar, vorzugsweise mindestens 0,3 bar, stärker bevorzugt mindestens 0,4 bar, insbesondere mindestens 0,5 bar, angelegt werden. Hierdurch kann die Befüllung beschleunigt werden und die Verdrängung des vorherigen Inhalts, im Fall des Befüllens der Filtervorrichtung mit dem Gas oder Gasgemisch in der Regel Luft und im Fall des Befüllens der Filtervorrichtung mit der wässrigen Lösung das Gas oder Gasgemisch, effizienter erfolgen, was die Effektivität des Befüllens erhöht.

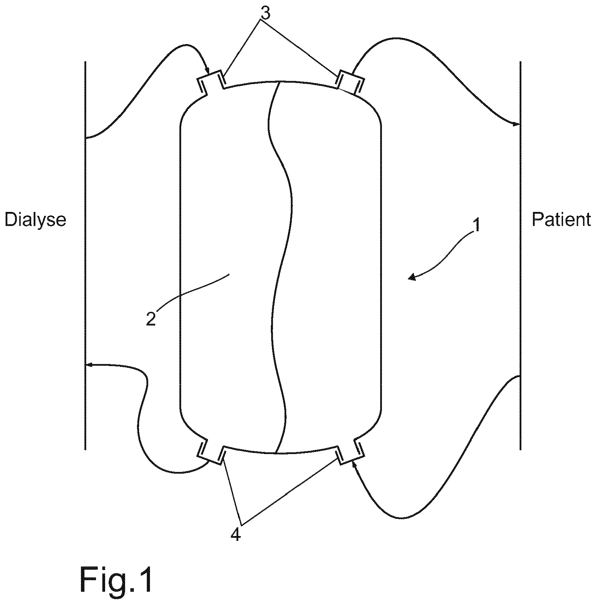

In einer bevorzugten Ausführungsform gemäß der Figur wird während des Befüllens der Filtervorrichtung 1 (dialyseseitiger Bereich 2) mit der wässrigen Lösung die Filtervorrichtung 1 auf eine Temperatur von weniger als 20 °C gekühlt und/oder die zum Befüllen der Filtervorrichtung 1 verwendete wässrige Flüssigkeit weist eine Temperatur von weniger als 20 °C auf. Hierdurch wird der Umstand ausgenutzt, dass die Löslichkeit von Gasen in Wasser umso größer ist, je niedriger die Temperatur ist, so dass sich das Gas bzw. das Gasgemisch bei einer Temperatur unter 20 °C besser und vollständiger löst, was zusätzlich die Effektivität des Befüllens erhöht. Insbesondere beträgt die Temperatur weniger als 18 °C, vorzugsweise weniger als 15 °C, stärker bevorzugt weniger als 10 °C, insbesondere weniger als 6 °C. Um einen übermäßigen Gegendruck auf Grund der Viskositätszunahme der wässrigen Lösung bei niedrigen Temperaturen zu vermeiden, beträgt die Temperatur vorzugsweise 1 °C oder mehr, stärker bevorzugt 2 °C oder mehr, insbesondere 4 °C oder mehr. Enthält die wässrige Lösung Ionen oder andere gelöste Stoffe, kann die Temperatur auch noch weiter abgesenkt werden.

Enthält das Gas oder Gasgemisch CO₂ oder ein anderes mit Wasser sauer reagierendes Gas, kann zur weiteren Erhöhung der Löslichkeit des Gases oder Gasgemisches in der wässrigen Lösung der pH-Wert der wässrigen Lösung erhöht werden, beispielsweise auf einen pH-Wert von 8 oder mehr, insbesondere 9 oder mehr. Hierdurch ist ein besonders schnelles und effektives Befüllen der Filtervorrichtung 1 möglich.

In einer Weiterbildung liegt die Flussrate der wässrigen Lösung während des Befüllens der Filtervorrichtung 1 mit der wässrigen Lösung über einer für die anschließende Behandlung vorgesehenen Flussrate bzw. den Flussraten durch die jeweiligen Kompartimente der Filtervorrichtung. Üblicherweise beträgt bei der Behandlung die Flussrate auf der Dialysatseite ca. 500 ml/min und auf der Blutseite ca. 250 bis 300 ml/min. Beim Befüllen der Filtervorrichtung 1 mit der wässrigen Lösung ist es vorteilhaft, wenn die Flussrate der Lösung mehr als 500 ml/min, insbesondere mehr als 750 ml/min beträgt. Hierdurch wird in kürzerer Zeit mehr frische Flüssigkeit durchgeleitet, die dementsprechend mehr von dem Gas oder Gasgemisch aufnehmen kann, so dass die Befüllung der Filtervorrichtung 1 schneller und effektiver erfolgen kann.

## Patentansprüche

1. Filtervorrichtung (1) zur selektiven Entfernung von Substanzen aus Blut oder anderen Körperflüssigkeiten, deren Innenraum, vorzugsweise deren reinigungsflüssigkeitsseitiger Bereich (2), mit einem Gas oder Gasgemisch gefüllt ist, das eine mindestens zweifach höhere Löslichkeit in Wasser als Luft aufweist,
**dadurch gekennzeichnet, dass**
der mit dem Gas oder Gasgemisch gefüllte Innenraum der Filtervorrichtung an der Innenseite des Gehäuses der Filtervorrichtung, vorzugsweise im Bereich des reinigungsflüssigkeitsseitigen Eingangs und/oder Ausgangs der Filtervorrichtung, zusätzlich mit einem hydrophilen Material zumindest teilweise versehen ist, um die Reaktionsfläche zur Lösung des Gases zu vergrößern.

2. Filtervorrichtung (1) nach Anspruch 1, wobei das Gas oder Gasgemisch eine im Vergleich zu Luft erhöhte Konzentration an CO₂ aufweist.

3. Filtervorrichtung (1) nach Anspruch 1 oder 2, wobei das Gas oder Gasgemisch mindestens 50 Vol-% CO₂ enthält.

4. Filtervorrichtung (1) nach einem der vorhergehenden Ansprüche, wobei es sich bei der Filtervorrichtung (1) um einen Plattendialysator handelt, in dem vorzugsweise schichtförmige Membrane angeordnet sind.

5. Filtervorrichtung (1) nach einem der Ansprüche 1 bis 3, wobei es sich bei der Filtervorrichtung (1) um einen Hohlfaserdialysator handelt, in dem vorzugsweise einzelne Hohlfaser-Membrane enthalten sind.

6. Filtervorrichtung (1) nach einem der Ansprüche 1 bis 3, wobei es sich bei der Filtervorrichtung (1) um eine Adsorberkartusche handelt.

7. Filtervorrichtung nach einem der vorhergehenden Ansprüche, wobei die Filtervorrichtung (1) gasdichte Verschlüsse (3, 4) aufweist und/oder gasdicht verpackt ist und wobei die Filtervorrichtung (1) steril vorliegt.

8. Verfahren zum Befüllen einer Filtervorrichtung (1) für Blut oder andere Körperflüssigkeiten mit einer wässrigen Lösung,
**dadurch gekennzeichnet, dass**
eine Filtervorrichtung (1) gemäß einem der Ansprüche 1 bis 7 mit der wässrigen Lösung befüllt wird.

9. Verfahren nach Anspruch 8, wobei während des Befüllens der Filtervorrichtung (1) mit dem Gas oder Gasgemisch und/oder während des Befüllens der Filtervorrichtung (1) mit der wässrigen Lösung ein Überdruck von mindestens 0,2 bar angelegt wird.

10. Verfahren nach Anspruch 8 oder 9, wobei während des Befüllens der Filtervorrichtung (1) mit der wässrigen Lösung die Filtervorrichtung (1) auf eine Temperatur von weniger als 20 °C gekühlt wird und/oder die zum Befüllen der Filtervorrichtung (1) verwendete wässrige Flüssigkeit eine Temperatur von weniger als 20 °C aufweist.

11. Verfahren nach einem der Ansprüche 8 bis 10, wobei die Flussrate der wässrigen Lösung während des Befüllens der Filtervorrichtung (1) mit der wässrigen Lösung über der für die anschließende Behandlung vorgesehene Flussrate liegt.

## Claims

1. Filter device (1) for selective removal of substances from blood or other body fluids, whose inner space, preferably whose region (2) on the cleaning fluid side is filled with a gas or gas mixture which has a solubility in water that is at least twice as high as that of air,
**characterized in that**
the inner space of the filter device filled with the gas or gas mixture is at least partially filled with a hydrophilic material in the region of the input and/or output on the cleaning fluid side of the filter device in order to enlarge the reaction surface for dissolving the gas.

2. Filter device (1) according to claim 1, wherein the gas or gas mixture has a higher concentration of CO₂ as compared to air.

3. Filter device (1) according to claim 1 or 2, wherein the gas or gas mixture comprises at least 50 vol-% CO₂.

4. Filter device (1) according to one of the preceding claims, wherein the filter device (1) is a plate dialyzer in which preferably layered membranes are arranged.

5. Filter device (1) according to one of the claims 1 to 3, wherein the filter device (1) is a hollow-fiber dialyzer in which preferably individual hollow-fiber membranes are comprised.

6. Filter device (1) according to one of the claims 1 to 3, wherein the filter device (1) is an adsorber cartridge.

7. Filter device according to one of the preceding claims, wherein the filter device (1) has gas-tight locks (3, 4) and/or is gas-tightly packed and wherein the filter device (1) is sterile.

8. Method for filling a filter device (1) for blood or other body fluids with an aqueous solution,
**characterized in that**
a filter device (1) according to one of the claims 1 to 7 is filled with the aqueous solution.

9. Method according to claim 8, wherein during filling of the filter device (1) with the gas or gas mixture and/or during filling of the filter device (1) with the aqueous solution, an excess pressure of at least 0.2 bar is applied.

10. Method according to claim 8 or 9, wherein during filling of the filter device (1) with the aqueous solution, the filter device (1) is cooled down to a temperature of less than 20 °C and/or the aqueous fluid used for filling the filter device (1) has a temperature of less than 20 °C.

11. Method according to one of the claims 8 to 10, wherein the flow rate of the aqueous solution during filling of the filter device (1) with the aqueous solution is higher than the flow rate intended for the subsequent treatment.

## Revendications

1. Dispositif de filtrage (1) servant à éliminer de manière sélective des substances du sang ou d'autres liquides corporels, dont l'espace intérieur, de préférence dont la zone (2) située du côté du liquide d'épuration, est remplie d'un gaz ou d'un mélange de gaz qui présente une solubilité dans l'eau au moins deux fois plus élevée que l'air,
**caractérisé en ce**
**que** l'espace intérieur rempli du gaz ou du mélange de gaz, du dispositif de filtrage, est pourvu au moins en partie, au niveau du côté intérieur du boîtier du dispositif de filtrage, de préférence dans la zone de l'entrée et/ou de la sortie, située du côté du liquide d'épuration, du dispositif de filtrage, en plus avec un matériau hydrophile afin d'agrandir la surface de réaction aux fins de la dissolution du gaz.

2. Dispositif de filtrage (1) selon la revendication 1, dans lequel le gaz ou le mélange de gaz présente une concentration en CO₂ plus importante en comparaison avec l'air.

3. Dispositif de filtrage (1) selon la revendication 1 ou 2, dans lequel le gaz ou le mélange de gaz contient au moins 50 % en vol. de CO₂.

4. Dispositif de filtrage (1) selon l'une quelconque des revendications précédentes, dans lequel le dispositif de filtrage (1) est un dialyseur à plaques, dans lequel sont disposées de préférence des membranes en forme de couche.

5. Dispositif de filtrage (1) selon l'une quelconque des revendications 1 à 3, dans lequel le dispositif de filtrage (1) est un dialyseur à fibres creuses, dans lequel sont contenues de préférence diverses membranes à fibres creuses.

6. Dispositif de filtrage (1) selon l'une quelconque des revendications 1 à 3, dans lequel le dispositif de filtrage (1) est une cartouche d'adsorbeur.

7. Dispositif de filtrage selon l'une quelconque des revendications précédentes, dans lequel le dispositif de filtrage (1) présente des systèmes de fermeture (3, 4) étanches aux gaz et/ou est conditionné de manière étanche aux gaz, et dans lequel le dispositif de filtrage (1) disponible est stérile.

8. Procédé servant à remplir un dispositif de filtrage (1) pour du sang ou d'autres liquides corporels avec une solution aqueuse,
**caractérisé en ce**
**qu'**un dispositif de filtrage (1) selon l'une quelconque des revendications 1 à 7 est rempli avec la solution aqueuse.

9. Procédé selon la revendication 8, dans lequel une surpression d'au moins 0,2 bar est appliquée au cours du remplissage du dispositif de filtrage (1) avec le gaz ou le mélange de gaz et/ou au cours du remplissage du dispositif de filtrage (1) avec la solution aqueuse.

10. Procédé selon la revendication 8 ou 9, dans lequel le dispositif de filtrage (1) est refroidi à une température inférieure à 20 °C au cours du remplissage du dispositif de filtrage (1) avec la solution aqueuse, et/ou dans lequel le liquide aqueux utilisé pour le remplissage du dispositif de filtrage (1) présente une température inférieure à 20 °C.

11. Procédé selon l'une quelconque des revendications 8 à 10, dans lequel le débit de la solution aqueuse au cours du remplissage du dispositif de filtrage (1) avec la solution aqueuse est supérieur au débit prévu pour le traitement qui suit immédiatement après.
